# EUROPEAN PATENT APPLICATION

(11) **EP 4 681 695 A1**
(43) Date of publication of application: **21.01.2026**
(21) Application number: 24200599.9
(22) Date of filing: 16.09.2024
(51) Int. Cl.: A61H 5/00

(54) **VIRTUAL REALITY-BASED VISUAL FUNCTION IMPROVEMENT SYSTEM**

(30) Priority: 18.07.2024 KR 20240094731
(71) Applicant: Jeonbuk National University Hospital, Jeonju-si Jeonbuk-do 54907 (KR); INDUSTRIAL COOPERATION FOUNDATION JEONBUK NATIONAL UNIVERSITY, Jeonju-si, Jeollabuk-do 54896 (KR)
(72) Inventor: LEE, Haeng-Jin, 54907 Jeonju-si, Jeonbuk-do (KR); KO, Myoung-Hwan, 54986 Jeonju-si, Jeonbuk-do (KR)
(74) Representative: Cabinet Chaillot

(57) **Abstract**

The present disclosure is directed to providing a visual function improvement system to treat amblyopia through "binocular stimulation amblyopia treatment" using a virtual three dimensional image.

This system includes a video headset with a means for allowing a user to wear the headset on the user's head, a separate display configured to display distinct visual stimulation images to the left and right eyes of the user in response to the user's gaze, and an image providing means that delivers visual stimulation videos or images to the separate display. The image providing means includes a binocular visual stimulation image providing unit, which separately presents a converted image to the amblyopic eye and a normal image to the fellow eye. By providing an unclear image to the fellow (or normal) eye, the amblyopic eye is activated, thereby improving the visual function imbalance between the two eyes.

## Description

### [Technical Field]

The present disclosure relates to a system for improving visual function, and more specifically, to a visual function improvement system using a virtual reality-based digital device, which performs "binocular stimulation amblyopia treatment" using a virtual image, wherein the visual function improvement system includes a video headset having a wearing means for allowing a user to wear a headset on a head, a separate display for displaying separated visual stimulation images to left and right eyes of the user, respectively, and an image providing means for providing a visual stimulation video or image to the separate display, wherein the image providing means includes a binocular visual stimulation image providing unit for separately providing a converted image and a normal image to the separate display so that an amblyopic eye is activated by providing a blurred image to a normal fellow eye among two eyes to improve visual function imbalance of the two eyes.

### [Background Art]

Visual function refers to the function of the eye, including binocular vision, such as vision, color vision and stereopsis. Vision is a complex function that is formed only through the accurate and balanced cooperation of various structures from the eyes to the cerebral cortex. Since these structures mature almost simultaneously, the development of each structure affects the development of the entire visual system. Some of these developmental processes proceed as planned before birth, but some are controlled by nerve cells activated by visual stimulation experienced after birth. In other words, at birth, 70% of the visual cortex contains binocular visual cortex neurons and the remaining monocular visual cortex neurons. After birth, if there is appropriate visual stimulation on both eyes, the binocular visual cortex neurons continue to differentiate and develop, and eventually acquire the functions of normal vision and stereopsis. Therefore, if appropriate visual stimulation is not received during childhood after birth, the development of vision is limited, resulting in low vision.

Amblyopia refers to the failure of visual functions such as vision to develop normally during the critical period of vision development. Vision may deteriorate in only one eye, or both eyes may be in a low state. Here, amblyopia refers to abnormal visual function in which corrected vision does not reach 20/20 even when wearing glasses that correct refractive errors. Amblyopia is the most common cause of vision loss in children and ranks fifth among the causes of low vision in adults. According to recent reports, approximately 10% of the pediatric population in the world is currently diagnosed with amblyopia, and the number is continuing to increase.

To solve the problem of amblyopia, glasses should be used for refractive errors, and if there is an accompanying eye disease that hinders vision development, the underlying disease should be treated. If visual function does not improve with glasses, etc., occlusion therapy or eye drop therapy should be attempted to improve the vision of the poor eye.

However, the occlusion treatment method of attaching an occluding patch to cover one eye, which is particularly used currently, is very similar to the method used about 80 years ago. Also, attaching an occluding patch to good eye has the disadvantages of very low compliance in pediatric patients and the long-term treatment, and it is difficult to predict the results. In addition, occlusion has little effect in adult patients with amblyopia. In addition, if amblyopia is discovered and treated early before the age of 8 to 10, vision improvement is possible, but if this period is missed, visual function can no longer be treated by any method, resulting in lifelong visual impairment, which is not only an individual inconvenience but also an economic burden on the family, society, and the country.

Furthermore, the existing occlusion treatment does not improve the binocular signal imbalance, which is another important amblyopia mechanism, because it is based on the principle of restoring monocular visual blockage among the mechanisms of amblyopia. Since amblyopia can lead to lifelong disability if the treatment period is missed, there is currently no treatment method that can be used for the 20-30% of pediatric patients who do not improve with occlusion treatment and eye drop treatment, and for adolescent or adult amblyopia patients who missed the period of amblyopia treatment. Therefore, a new concept of treatment must be developed, and "binocular stimulation treatment" is currently emerging as the only method.

The principle of "binocular stimulation treatment" is as follows.

Normally, due to the difference in vision between two eyes, a blurred image is input to the amblyopic eye, and a clear image is input to the normal eye, into the visual cortex that is the occipital lobe of the brain. Afterwards, an inhibitory signal is sent from the normal eye to the amblyopic eye, and as a result, the amblyopic eye is further inhibited, which limits the development of vision.

However, in the case of "binocular stimulation treatment", if the visual stimulation of two eyes is separated so that the normal eye is blurred to reduce the visual input to the normal fellow eye to the level of amblyopia, the inhibitory signal sent to the amblyopic eye is reduced, resulting in an improvement in the vision of the amblyopic eye.

In other words, "binocular stimulation treatment" is an amblyopia treatment method that effectively removes the inhibitory signals transmitted from the normal eye to the amblyopic eye, allowing both eyes to develop simultaneously.

However, although "binocular stimulation treatment" is being studied and developed as medical devices both domestically and internationally, only primary treatment for binocular stimulation treatment is being performed, and research on methods utilizing medical devices using virtual reality is insufficient, and research on providing various visual stimulations to help patients focus has not been conducted. In particular, a visual stimulation method for creating a visual function state such as amblyopic eye, which makes it difficult to see well, has not been discovered yet, and a method for monitoring while tracking eyegaze in a three-dimensional image has also not been discovered.

### [RELATED LITERATURES]

### [Patent Literature]

Korean Unexamined Patent Publication No. 10-2024-0008500

### [Disclosure]

### [Technical Problem]

Accordingly, the inventors of the present disclosure have comprehensively considered all the above-mentioned matters and, at the same time, made great efforts to develop a new structural visual function improvement system with the idea of solving the technical limitations and problems of the existing technology for treating amblyopia, and as a result, has designed the present disclosure.

Therefore, the technical challenge and purpose that the present disclosure seeks to solve is to overcome the problems and limitations of an occlusion treatment method of attaching an occluding patch to cover one eye in the existing treatment of amblyopia, and to provide a binocular stimulation treatment, which is being recently studied, to patients more effectively and to increase the convenience of the patients.

The technical problems and purposes that the present disclosure seeks to solve are not limited to the technical problems and purposes mentioned above, and those skilled in the art will be able to clearly understand other technical problems and purposes not mentioned from the description below.

### [Technical Solution]

The specific means of implementing the present disclosure to effectively achieve a specific technical purpose while specifying a new idea to solve the technical challenges of the present disclosure as described above provides a video headset having a wearing means for allowing a user to wear a headset on a head; a separate display configured to display separated visual stimulation images to left and right eyes of the user, respectively; and an image providing means configured to provide a visual stimulation video or image to the separate display, wherein the image providing means includes a binocular visual stimulation image providing unit configured to separately provide a converted image and a normal image to the separate display so that an amblyopic eye (with relatively weak vision) is activated by providing an unclear image to a fellow eye (with normal vision) among two eyes to improve visual function imbalance of the two eyes. Preferably, the image providing means includes a responsive content image providing unit configured to track the gaze of the user through pupil detection means and provide a content image that operates in response to the gaze of the user to be separated into as a converted image and a normal image. Preferably, the image providing means includes a three-dimensional virtual image providing unit to provide a three-dimensional virtual image, and a three-dimensional gaze line display configured to analyze the gaze of the user and additionally display a focus virtual three-dimensional gaze line toward a target object on the virtual image when the user looks at the target object on the virtual image.

### [Advantageous Effects]

According to the technology of the present disclosure, which is based on a unique solution to solve the above technical problems, the present disclosure has the effect of improving the visual function of the user by activating the amblyopic eye by providing a converted image and a normal image on a separate display and using a virtual image. In addition, the present disclosure increases the concentration and participation of the user by providing a responsive content image providing unit, so that visual function treatment is performed more smoothly. Furthermore, it is possible to monitor eye movements and visual function treatment in real time by tracking the gaze of both eyes, and the present disclosure has the effect of improving the concentration of visual function treatment by providing a three-dimensional gaze line to the user so that the user may confirm three-dimensional information including the depth of the gaze of the user in real time.

The effects of the present disclosure are not limited to the effects mentioned above, and other effects not mentioned herein will be clearly understood by those skilled in the art from the description of the claims.

### [Description of Drawings]

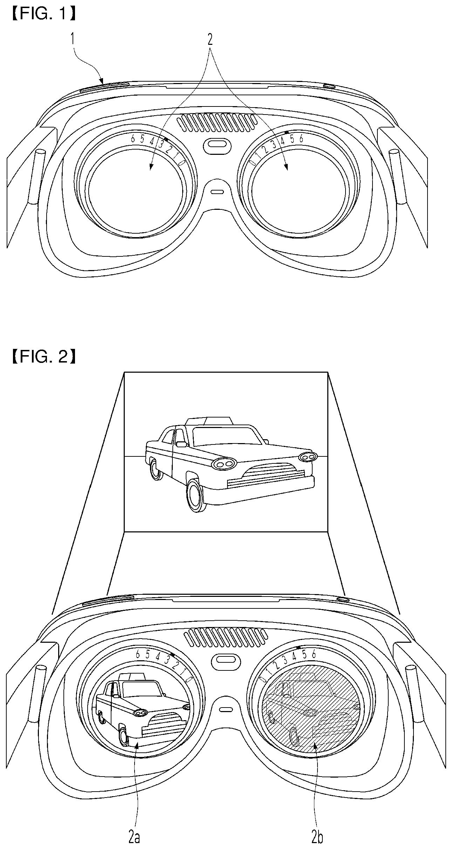

FIG. 1 is a diagram exemplarily showing an example of a video headset to which an embodiment of the present disclosure is applied.
FIG. 2 is a diagram exemplarily showing an example of displaying a normal image and a brightness change image on a separate display using a brightness change image providing unit according to the present disclosure.

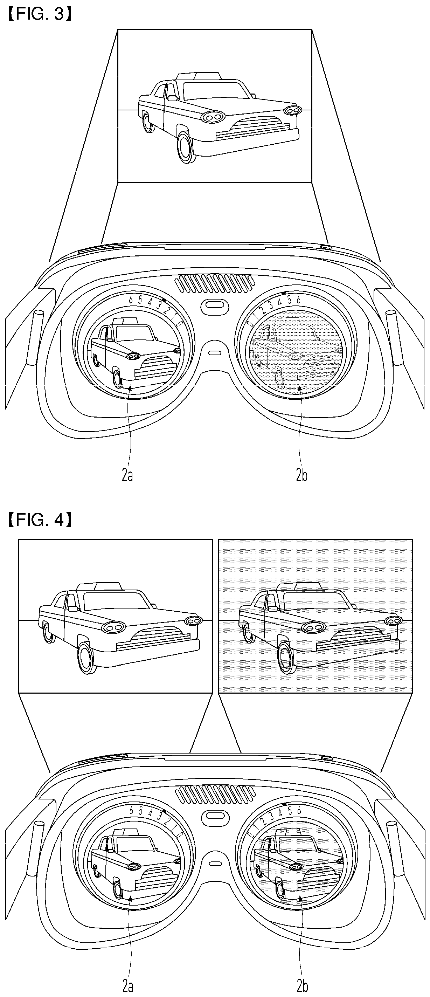

FIG. 3 is a diagram exemplarily showing an example of displaying a normal image and a chroma change image on the separate display using a chroma change image providing unit according to the present disclosure.
FIG. 4 is a diagram exemplarily showing an example of displaying a normal image and a focus change image on the separate display using a focus change image providing unit according to the present disclosure.

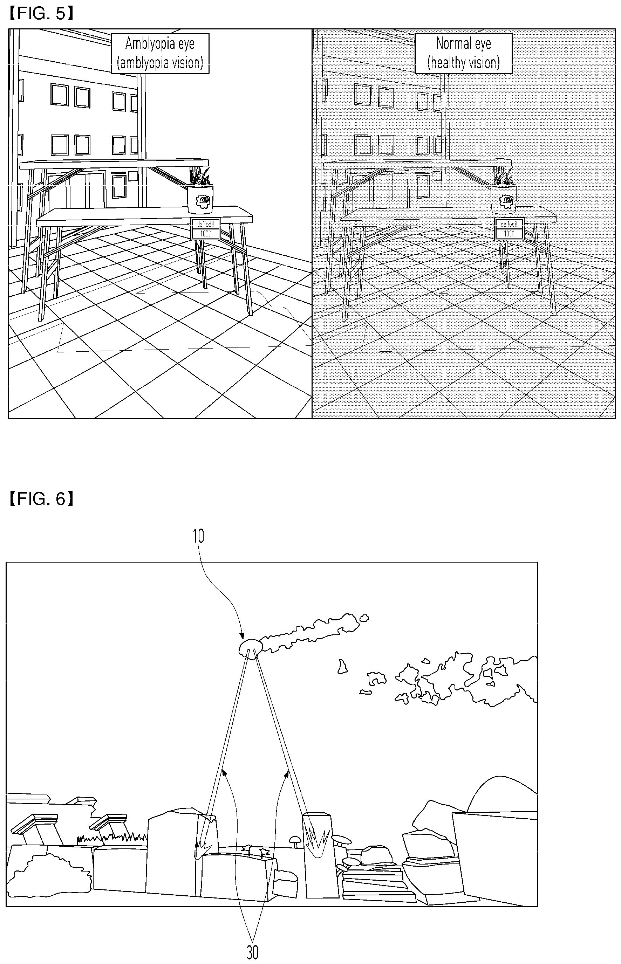

FIG. 5 is a diagram exemplarily showing an example of displaying a content image on the separate display using a responsive content image providing unit according to the present disclosure.

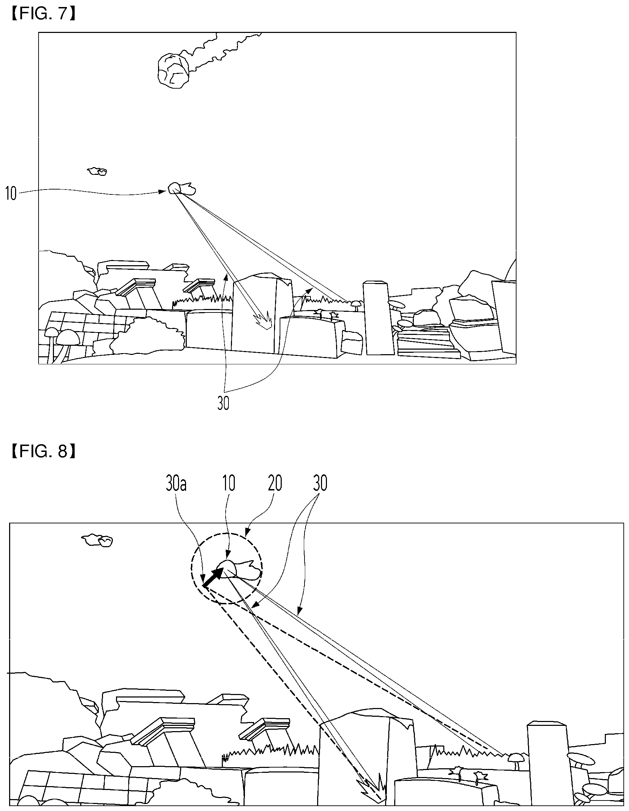

FIGS. 6 and 7 are diagrams exemplarily showing the appearance of displaying a three-dimensional gaze line display in three dimensions according to the gaze of the user when displaying an image on the separate display using a three-dimensional virtual image providing unit according to the present disclosure.
FIG. 8 is a diagram exemplarily showing the appearance of displaying a gaze line display stably using a target acceptance area processing unit when the gaze of the user stays in a target acceptance area, in displaying an image on the separate display using a three-dimensional virtual image providing unit according to the present disclosure.

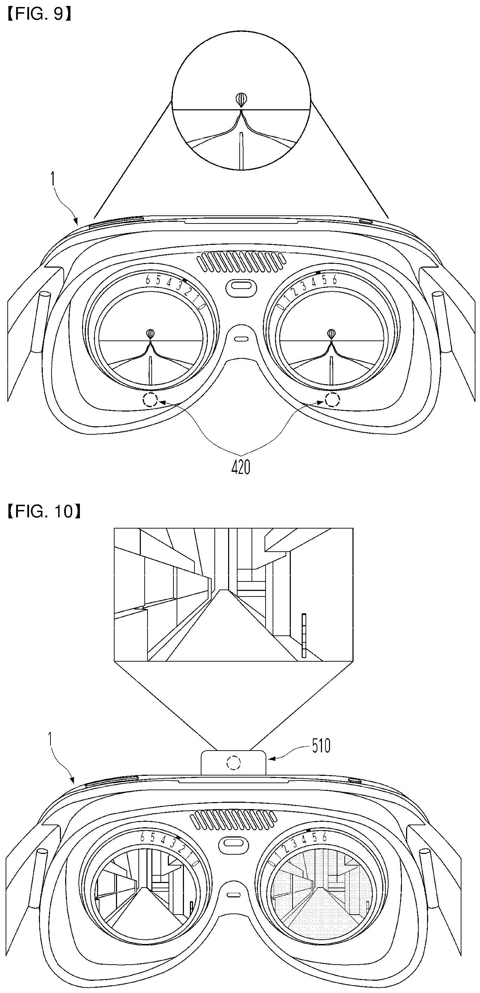

FIG. 9 is a diagram exemplarily showing the appearance of an imbalance confirmation image providing unit according to the present disclosure.
FIG. 10 is a diagram exemplarily showing the appearance of a real-time video conversion providing unit equipped with a real-time video capturing unit according to the present disclosure.

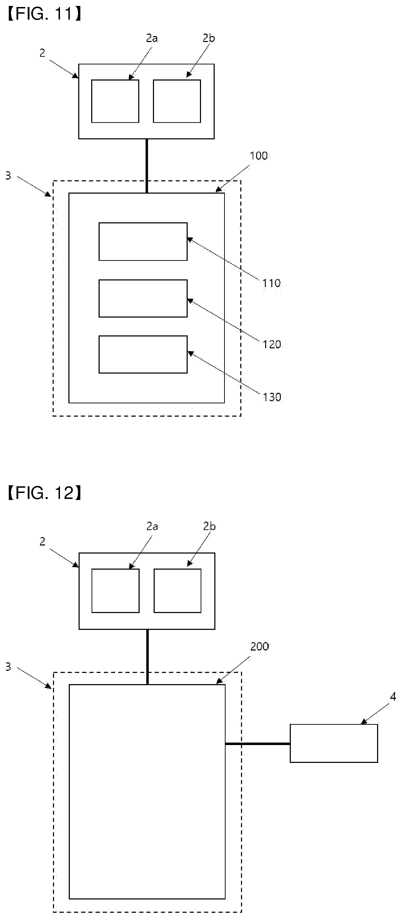

FIG. 11 is a diagram schematically showing the relationship of a binocular visual stimulation image providing unit according to the present disclosure.
FIG. 12 is a diagram schematically showing the relationship of a responsive content image providing unit according to the present disclosure.

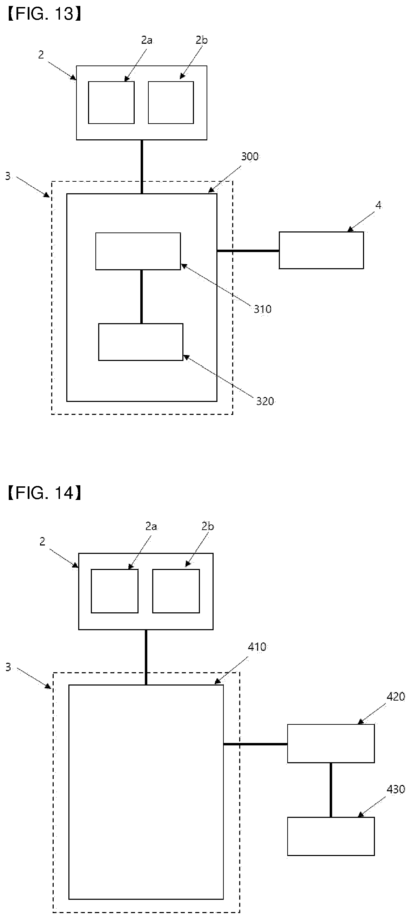

FIG. 13 is a diagram schematically showing the relationship of a three-dimensional virtual image providing unit according to the present disclosure.
FIG. 14 is a diagram schematically showing the relationship of an imbalance confirmation image providing unit according to the present disclosure.

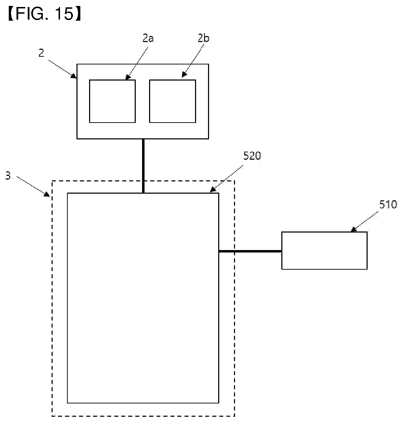

FIG. 15 is a diagram schematically showing the relationship of a real-time video conversion providing unit according to the present disclosure.

### [Best Mode]

Hereinafter, embodiments according to the present disclosure will be described in more detail with reference to the attached drawings. Prior to explaining the present disclosure, it should be noted that the terms described below have been defined in consideration of their functions in the present disclosure, and should be interpreted as concepts that conform to the technical idea of the present disclosure and meanings commonly used or recognized in the relevant technical field. In addition, if a specific description of a known function or configuration related to the present disclosure is judged to obscure the gist of the present disclosure, the detailed description will be omitted.

The drawings attached hereto illustrate the configuration and operation of the technology and are exaggerated or simplified for convenience and clarity of understanding, and it should be noted that each component does not exactly match the actual size and shape.

In addition, the term "and/or" in this specification means a combination of a plurality of related described items or including any item among a plurality of related described items, and when it is said that a certain part includes a certain component, this does not mean that other components are excluded, but rather that other components can be further included, unless specifically stated otherwise.

That is, terms such as "include," "have," "configure," and "comprise" indicate the presence of a feature, number, step, operation, component, part, or combination thereof described in this specification, but should be understood to not exclude the possibility of the presence or addition of one or more other features, numbers, steps, operations, components, parts, or combinations thereof.

Also, the terms "top", "bottom", "upper surface", "lower surface", "upper", "lower", "upper side", "lower side", "front/back", "left/right", etc. are used for convenience to distinguish the relative positions of each component. For example, the upper part of a drawing may be named or referred to as the lower part, and the upper part may be named or referred to as the lower part. Also, the length direction may be named or referred to as the front/back direction, and the width direction may be named or referred to as the left/right direction.

Also, the terms "first", "second", etc. may be used to describe various components. That is, the terms "first", "second", etc. may be used only for the purpose of distinguishing one component from another. For example, the first component may be referred to as the second component, and the second component may also be referred to as the first component, as long as it does not exceed the scope of protection of the present disclosure.

The present disclosure is directed to providing a visual function improvement system to treat amblyopia to provide "binocular stimulation amblyopia treatment" using a virtual image. The terms and words used in this specification and claims should not be interpreted as limited to their usual or dictionary meanings, and should be interpreted as meanings and concepts that conform to the technical idea of the present disclosure based on the principle that an inventor can appropriately define the concept of a term in order to explain his or her own invention in the best manner.

A visual function improvement system according to the present disclosure includes a video headset 1 having a wearing means for allowing a user to wear a headset on a head, a separate display 2 configured to display separated visual stimulation images to left and right eyes of the user, respectively, and an image providing means 3 configured to provide a visual stimulation video or image to the separate display.

Here, the image providing means 3 includes a binocular visual stimulation image providing unit 100 configured to separately provide a converted image and a normal image to the separate display so that an amblyopic eye (with relatively weak vision) is activated by providing an unclear image to a fellow eye (with normal vision) among two eyes to improve visual function imbalance of the two eyes.

At this time, the binocular visual stimulation image providing unit 100 includes a brightness change image providing unit 110, a chroma change image providing unit 120, or a focus change image providing unit 130.

Here, the brightness change image providing unit 110 is configured to provide a normal image to an amblyopia display and provide an image with brightness changed in stages to a healthy vision display, the chroma change image providing unit 120 is configured to provide a normal image to the amblyopia display and provide an image with chroma changed in stages to the healthy vision display, and the focus change image providing unit 130 is configured to provide a normal image to the amblyopia display and provide an image with a focus changed in stages to the healthy vision display.

In addition, the visual function improvement system includes a pupil detection means 4 provided on one side of the video headset to detect an eye of the user to recognize a gaze of the user.

In addition, the image providing means 3 includes a responsive content image providing unit 200 configured to track the gaze of the user through pupil detection means and provide a content image that operates in response to the gaze of the user to be separated into as a converted image and a normal image,

In addition, the image providing means 3 includes a three-dimensional virtual image providing unit 300 configured to provide a three-dimensional virtual image to the user as a left and right parallax image on the separate display.

Moreover, the image providing means 3 includes a three-dimensional gaze line display 310 configured to analyze the gaze of the user through an information value of the pupil detection means 4 and additionally display a focus virtual three-dimensional gaze line toward a target object on the virtual image provided by the three-dimensional virtual image providing unit 300 when the user looks at the target object on the virtual image.

At this time, the image providing means 3 includes a target acceptance area processing unit 320 configured to adjust and display a three-dimensional gaze line toward the target object when the gaze of the user enters an acceptance area surrounding the target object, even if the gaze of the user is not accurately fixed on the target object, so that the three-dimensional gaze line is stably displayed on the target object.

In addition, the visual function improvement system includes a visual function imbalance confirmation means configured to confirm the visual function imbalance of the user by distinguishing a relative healthy eye and an amblyopia eye, and measure an imbalance value,

Here, the visual function imbalance confirmation means includes an imbalance confirmation image providing unit 410 configured to provide an imbalance confirmation image on the separate display, an eye measurement unit 420 configured to measure two eyes (refractive index, pupil change value, pupil shift value) of the user who looks at the imbalance confirmation image displayed on the separate display, and an imbalance calculation unit 430 configured to calculate the degree of imbalance of the two eyes through a value of the eye measurement unit,

In addition, the visual function improvement system includes a real-time image capturing unit 510 configured to capture an external image in real time on one side of the video headset to convert a real-time external image and provide the real-time external image as a visual stimulation image, and a real-time video conversion providing unit 520 configured to convert the external image captured by the real-time image capturing unit in real time to be separately provided as a converted external image and a normal external image on the separate display, respectively.

Meanwhile, the present disclosure is not limited to the above-described embodiment and the attached drawings, and may be modified and applied in various ways not illustrated within the scope that does not depart from the technical idea of the present disclosure, and it is obvious to a person having ordinary skill in the technical field to which the present disclosure belongs that each component can be replaced and changed to other equivalent embodiments and applied widely. Therefore, the contents related to modifying and applying the technical features of the present disclosure should be interpreted as being included within the technical idea and scope of the present disclosure.

**[Reference Symbols]**

| | | | |
|---|---|---|---|
| 1: | video headset | 2: | separate display |
| 2a: | amblyopia display | 2b: | healthy vision display |
| 3: | image providing means | 4: | pupil detection means |
| 10: | target object | 20: | acceptance area |
| 30: | three-dimensional gaze line | 30a: | actual gaze of user |
| 100: | binocular visual stimulation image providing unit | 110: | brightness change image providing unit |
| 120: | chroma change image providing unit | 130: | focus change image providing unit |
| 200: | responsive content image providing unit | | |
| 300: | three-dimensional virtual image providing unit | | |
| 310: | three-dimensional gaze line display | 320: | target acceptance area processing unit |
| 410: | imbalance confirmation image providing unit | 420: | eye measurement unit |
| 430: | imbalance calculation unit | | |
| 510: | real-time video conversion providing unit | 520: | real-time video conversion unit |

## Claims

1. A visual function improvement system using a virtual reality-based digital device, comprising:
a video headset (1) having a wearing means for allowing a user to wear a headset on a head;
a separate display (2) configured to display separated visual stimulation images to left and right eyes of the user, respectively; and
an image providing means (3) configured to provide a visual stimulation video or image to the separate display,
wherein the image providing means includes a binocular visual stimulation image providing unit (100) configured to separately provide a converted image and a normal image to the separate display so that an amblyopic eye (with relatively weak vision) is activated by providing an unclear image to a fellow eye (with normal vision) among two eyes to improve visual function imbalance of the two eyes.

2. The visual function improvement system using a virtual reality-based digital device according to claim 1,
wherein the binocular visual stimulation image providing unit (100) includes a brightness change image providing unit (110), a chroma change image providing unit (120), or a focus change image providing unit (130),
wherein the brightness change image providing unit (110) is configured to provide a normal image to an amblyopia display and provide an image with brightness changed in stages to a healthy vision display,
wherein the chroma change image providing unit (120) is configured to provide a normal image to the amblyopia display and provide an image with chroma changed in stages to the healthy vision display, and
wherein the focus change image providing unit (130) is configured to provide a normal image to the amblyopia display and provide an image with a focus changed in stages to the healthy vision display.

3. The visual function improvement system using a virtual reality-based digital device according to claim 1, further comprising:
a pupil detection means (4) provided on one side of the video headset to detect an eye of the user to recognize a gaze of the user,
wherein the image providing means includes a responsive content image providing unit (200) configured to track the gaze of the user through pupil detection means and provide a content image that operates in response to the gaze of the user to be separated into as a converted image and a normal image,
wherein the image providing means includes:
a three-dimensional virtual image providing unit (300) configured to provide a three-dimensional virtual image to the user as a left and right parallax image on the separate display;
a three-dimensional gaze line display (310) configured to analyze the gaze of the user through an information value of the pupil detection means and additionally display a focus virtual three-dimensional gaze line toward a target object on the virtual image provided by the three-dimensional virtual image providing unit when the user looks at the target object on the virtual image; and
a target acceptance area processing unit (320) configured to adjust and display a three-dimensional gaze line toward the target object when the gaze of the user enters an acceptance area surrounding the target object, even if the gaze of the user is not accurately fixed on the target object, so that the three-dimensional gaze line is stably displayed on the target object.

4. The visual function improvement system using a virtual reality-based digital device according to claim 1, further comprising:
a visual function imbalance confirmation means configured to confirm the visual function imbalance of the user by distinguishing a relative normal fellow eye and an amblyopic eye, and measure an imbalance value,
wherein the visual function imbalance confirmation means includes:
an imbalance confirmation image providing unit (410) configured to provide an imbalance confirmation image on the separate display;
an eye measurement unit (420) configured to measure two eyes of the user who looks at the imbalance confirmation image displayed on the separate display; and
an imbalance calculation unit (430) configured to calculate the degree of imbalance of the two eyes through a value of the eye measurement unit,
wherein the visual function improvement system further comprises:
a real-time image capturing unit (510) configured to capture an external image in real time on one side of the video headset to convert a real-time external image and provide the real-time external image as a visual stimulation image, and
a real-time video conversion providing unit (520) configured to convert the external image captured by the real-time image capturing unit in real time to be separately provided as a converted external image and a normal external image on the separate display, respectively.
